# EUROPEAN PATENT SPECIFICATION

(11) **EP 3 478 201 B1**
(45) Date of publication and mention of the grant of the patent: **01.01.2020**
(21) Application number: 17739509.2
(22) Date of filing: 30.06.2017
(51) Int. Cl.: A61B 18/20

(54) **A HAIR CUTTING DEVICE**
HAARSCHNEIDEVORRICHTUNG
DISPOSITIF DE COUPE DE CHEVEUX

(30) Priority: 30.06.2016 EP 16177339
(43) Date of publication of application: 08.05.2019
(73) Proprietor: Koninklijke Philips N.V., 5656 AE Eindhoven (NL)
(72) Inventor: THUMMA, Kiran, Kumar, 5656 AE Eindhoven (NL); JOHNSON, Mark, Thomas, 5656 AE Eindhoven (NL); BOURQUIN, Yannyk, Parulian, Julian, 5656 AE Eindhoven (NL); MOESKOPS, Bastiaan, Wilhelmus, Maria, 5656 AE Eindhoven (NL); VERHAGEN, Rieko, 5656 AE Eindhoven (NL)
(74) Representative: Uittenbroek, Arie Leendert
(86) International application number: PCT/EP2017/066303
(87) International publication number: WO 2018/002317

(56) References cited:
- WO-A1-2014/143670
- WO-A1-2014/197667
- WO-A1-2015/089425
- WO-A2-2008/115899
- GB-A- 2 447 366
- US-A1- 2016 067 734

## Description

### FIELD OF THE INVENTION

The invention relates to a hair cutting device for cutting (e.g. shaving) hair on a body of a subject, and in particular relates to a hair cutting device that uses laser light to cut or shave hair.

### BACKGROUND OF THE INVENTION

Shaving devices for cutting or shaving hair on a body of a subject typically make use of one or more blades that cut hairs as the blade is moved across the skin of the subject. The blades can be static within the device, for example as in a wet razor, whereas in other types of devices, for example electric shavers, one or more blade elements can be actuated (e.g. rotated or oscillated) in order to produce a cutting action.

However, an alternative type of shaving device has been proposed in WO 2014/143670 that makes use of laser light. In particular a laser light source is provided that is configured to generate laser light having a wavelength selected to target a predetermined chromophore to effectively cut a hair shaft. A fiber optic is located on a shaving portion of the device that is positioned to receive the laser light from the laser light source at a proximal end, conduct the laser light from the proximal end toward a distal end, and emit the light out of a cutting region of the fiber optic and toward hair when the cutting region is brought in contact with the hair.

### SUMMARY OF THE INVENTION

To achieve good shaving closeness, the cutting element of the shaving device (i.e. the fiber optic in the case of the device in WO 2014/143670) needs to be brought very close to the skin or even touch the skin. That device is made in such way that the light couples into a hair when a hair is in contact with the fiber optic. In particular, this is achieved by the core of the fiber optic having a lower refractive index than hair.

In order to cut hair effectively, the fiber optic should ideally remain in contact with the same part of the hair for a prolonged period of time to allow coupled light to initiate the coupling and cut through the minimum amount of hair. However, since the refractive index of skin is close to the refractive index of hair, the laser light will also be able to couple into the skin if the cutting element is brought into contact with the skin, and potentially burn or irritate the skin, particularly if the fiber optic remains on the same point of the skin for a prolonged period of time.

Therefore there is a need for an improved hair cutting device that reduces the risk of damage or injury to the skin of the subject.

According to a first aspect, there is provided a hair cutting device for cutting hair on a body of a subject, the hair cutting device comprising a light source for generating laser light at one or more specific wavelengths corresponding to wavelengths absorbed by one or more chromophores in or on hair; and a cutting element that comprises an optical waveguide that is coupled to the light source to receive laser light, wherein a portion of a sidewall of the optical waveguide forms a cutting face for contacting hair; wherein at least a portion of the cutting face is provided with a first coating or layer that is formed from or comprises a lipophilic material; and wherein at least a portion of the sidewall of the optical waveguide that can contact the skin of the subject has a second coating or layer that is formed from or comprises a lipophobic material.

The first coating or layer is provided to increase friction (e.g. provide a high friction) between the optical waveguide and hair. The second coating or layer is provided to reduce friction (i.e. provide a low friction) between the optical waveguide and skin.

In some embodiments, the lipophilic material is an oil, a heavy hydro-carbon, a fat, an ester, a petroleum by-product or derived product such as polyethene, polypropane, amino acids, or nickel silicon carbide.

In some embodiments, the first coating or layer is transparent or partially transparent to light at one or more of said one or more specific wavelengths. This enables light from the optical waveguide to pass into hair.

In some embodiments, the first coating or layer has a thickness between 0.01 microns and 100 microns, or a thickness between 0.1 microns and 10 microns.

In some embodiments, the first coating or layer has a refractive index that is equal to or higher than that of the optical waveguide and a refractive index that is equal to or lower than that of hair. This enables light from the optical waveguide to couple into hair.

In some embodiments, the lipophobic material is a polymer material. In some embodiments, the lipophobic material is polytetrafluoroethylene (PTFE) or a fluorinated compound.

In some embodiments, the second coating or layer is reflective to light at one or more of said one or more specific wavelengths. This helps to reduce the amount of light that can pass from the optical waveguide to the skin.

In some embodiments, the second coating or layer has a thickness between 0.1 microns and 2000 microns, or a thickness between 0.1 microns and 500 microns.

In some embodiments, the second coating or layer has a refractive index that is lower than that of the optical waveguide. This helps to stop light from coupling from the optical waveguide into the skin.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a block diagram of a hair cutting device according to an embodiment of the invention;
Fig. 2 is a pair of schematic drawings showing different views of an exemplary hair cutting device according to an embodiment of the invention;
Fig. 3 is a graph illustrating the refractive index of hair; and
Fig. 4 is an illustration of an optical fibre cutting element according to a specific embodiment.

### DETAILED DESCRIPTION OF THE EMBODIMENTS

As noted above, the present invention provides an improvement in the safety and comfort of a subject that is using a laser light-based shaving device, for example as described in WO 2014/143670. In particular, it has been recognised that to prolong a time period of contact between the optical waveguide and hair whilst reducing a time period of contact between the optical waveguide and the skin components are incorporated into the shaving device, and specifically onto the optical waveguide, which increase the friction between the optical waveguide and the hair (e.g. which adhere or are 'sticky' to hair) and incorporate components onto the optical waveguide that decrease the friction between the optical waveguide and the skin (e.g. which do not adhere so much or are less 'sticky' to skin).

It will be appreciated that the invention is applicable to shaving devices (e.g. razors or electric shavers), and any other type of device that is used to cut hair (e.g. hair clippers), even if those devices do not necessary aim to provide a 'clean shave' (i.e. to remove hair at the level of the skin).

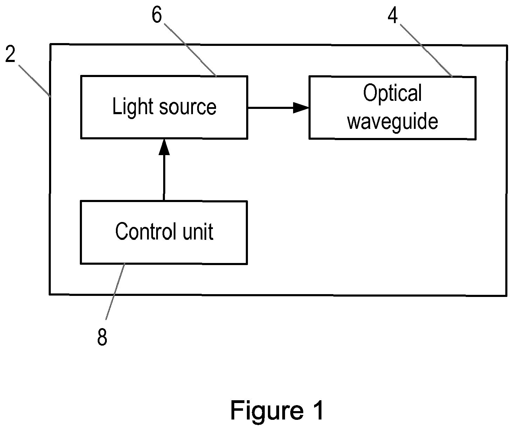

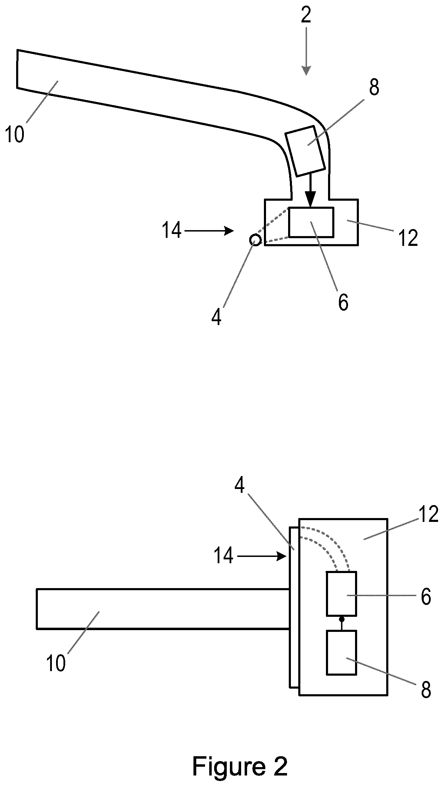

Fig. 1 is a block diagram of a hair cutting device 2 according to an embodiment of the invention. Fig. 2 shows a hair cutting device 2 in the form of a handheld razor according to an exemplary embodiment of the invention. The hair cutting device 2 is for cutting (e.g. shaving) hair on a body of a subject. The subject may be a person or an animal. The hair may be facial hair (i.e. hair on the subject's face), or hair on the subject's head or other part of their body (legs, chest, etc.).

The hair cutting device 2 comprises a cutting element 4 that enables hair to be cut as the hair cutting device 2 is moved over the skin of a subject. The cutting element 4 is an optical waveguide 4 that is arranged on the hair cutting device 2 so that the optical axis of the optical waveguide 4 (i.e. the line along which light typically propagates through the optical waveguide 4) is generally perpendicular to the direction in which the hair cutting device 2 is moved so that hairs contact the side wall of the optical waveguide 4 (the side wall corresponding to the long edge of the optical waveguide 4) as the hair cutting device 2 is moved across the skin of the subject. In some embodiments, the optical waveguide 4 is an optical fibre, although those skilled in the art will be aware of other types of optical waveguide that can be used according to the invention, such as a slab waveguide, a strip waveguide or a photonic crystal waveguide. An optical fibre comprises a core, and in some embodiments also comprises a cladding, which may or may not fully encompass the core (e.g. part of the core may be exposed).

A light source 6 is provided in the hair cutting device 2 that generates laser light at one or more specific wavelengths. The light source 6 is optically coupled to the optical waveguide 4 so that the laser light generated by the light source 6 is coupled into the optical waveguide 4 (and specifically coupled into an end of the optical waveguide 4 so that the laser light propagates through the optical waveguide 4).

The light source 6 is configured to generate laser light at one or more specific wavelengths that can be used to cut or burn through hair. In particular, each wavelength corresponds to the wavelength of light absorbed by a chromophore that is found in or on hair. As is known, a chromophore is the part of a molecule that provides the molecule with its colour. Thus, the laser light will be absorbed by the chromophore and converted into heat which will melt or burn the hair or otherwise destroy the bonds in the molecules of the hair, and it is this melting or burning that provides the cutting action of the hair cutting device 2.

Suitable chromophores that can be targeted by the laser light generated by the light source 6 include, but are not limited to, melanin, keratin and water. Suitable wavelengths of laser light that can be used include, but are not limited to, wavelengths selected from the range 380 nm (nanometers) to 500 nm and 2500 nm to 3500 nm. Those skilled in the art will be aware of the wavelengths of light that are absorbed by these chromophores, and thus also the specific wavelengths of light that the light source 6 should generate for this purpose, and further details are not provided herein.

In some embodiments the light source 6 can be configured to generate laser light at a plurality of wavelengths (either simultaneously or sequentially), with each wavelength being selected to target a different type of chromophore. This can improve the cutting action of the optical waveguide 4 since multiple types of molecules in the hair may be burnt using the laser light. Alternatively multiple light sources 6 can be provided that each generate laser light at a respective wavelength, and each light source 6 can be coupled to a respective optical waveguide 4 to provide multiple cutting elements 4 in the device 2.

The hair cutting device 2 also comprises a control unit 8 that controls the operation of the hair cutting device 2, and in particular is connected to the light source 6 to control the activation and deactivation of the light source 6 (and in some embodiments control the wavelength and/or intensity of the light generated by the light source 6). The control unit 8 may activate and deactivate the light source 6 in response to an input from a user of the hair cutting device 2. The control unit 8 can comprise one or more processors, processing units, multi-core processors or modules that are configured or programmed to control the hair cutting device 2.

As noted above, Fig. 2 shows a hair cutting device 2 that is in the form of a handheld wet razor. Fig. 2 shows a side view and a bottom view of the razor 2. The razor 2 comprises a handle 10 for the subject (or other user of the device 2) to hold, and a head portion 12 that includes the cutting element 4 (optical waveguide/fibre). As shown, the optical waveguide 4 is arranged along an edge of the head portion, and a part of the optical waveguide 4 forms (or corresponds to) a cutting face 14. The cutting face 14 is the part of the optical waveguide 4 that is intended to come into contact with hair as the hair cutting device 2 is moved across the skin of the subject. A light source 6 and control unit 8 are shown as being incorporated into the head portion 12 and handle 10 respectively, but it will be appreciated that the positions of these components in the hair cutting device 2 as shown in Fig. 2 is not limiting. Likewise it will be appreciated that the embodiment shown in Fig. 2 is merely an example, and the invention can be incorporated or used in any type of hair cutting device 2 that conventionally comprises a blade for physically cutting or slicing hair (whether the blade is static or actuated in order to achieve a cutting action).

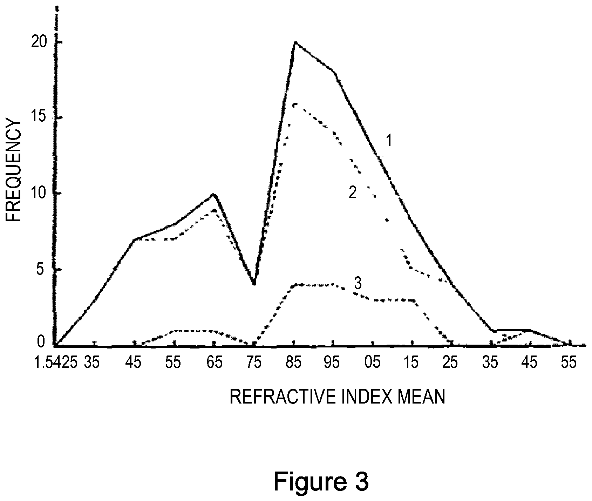

The graph in Fig. 3 illustrates the refractive index of hair, which can be found in a paper by M. D. Greenwell, A. Willner, Paul L. Kirk: Human Hair Studies: III. Refractive Index of Crown Hair, 31 Am. Inst. Crim. L. & Criminology 746 (1940-1941). Curve 1 is a composite line, curve 2 is a line representing the refractive index for Caucasian people, and curve 3 is a line representing the refractive index for non-Caucasian people. Thus, it can be seen that the refractive index of hair is between (approximately) 1.545 and 1.555, although there will be variation between individuals. For example the above paper also recognises that the refractive index of hair can depend on the sex of the subject, e.g. the refractive index of hair on a female is generally higher than the refractive index of hair on a male.

As is known, the optical waveguide 4 acts as a waveguide for the light coupled from the light source 6 through the occurrence of total internal reflection, since the refractive index of air is lower than that of the optical waveguide 4. However, if an object that has a refractive index higher than the optical waveguide 4 is put into contact with the optical waveguide 4, then the total internal reflection is 'frustrated' and light can couple from the optical waveguide 4 into that object. Thus, in order for light to be coupled into a hair from the optical waveguide 4 (to provide the cutting action according to the invention), the optical waveguide 4 must have the same or a lower refractive index than hair at the point at which the hair contacts the optical waveguide 4. Thus, the optical waveguide 4 must have the same or a lower refractive index than hair at least at the cutting face 14 portion of the optical waveguide 4. Preferably the refractive index of the optical waveguide 4 at the cutting face 14 is the same as that of hair since that provides the best coupling of light from the optical waveguide 4 to the hair.

Thus, in some embodiments, the refractive index of the optical waveguide 4 at least at the cutting face 14 is equal to or lower than 1.56. More preferably the refractive index of the optical waveguide 4 at least at the cutting face 14 is equal to or lower than 1.55. Even more preferably, the refractive index of the optical waveguide 14 at least at the cutting face 14 is equal to or lower than 1.54, since this refractive index is below the refractive indices identified in Fig. 3.

In some embodiments, a lower bound for the refractive index of the optical waveguide 4 at the cutting face 14 can be 1.48, 1.51, 1.53 or 1.54.

A range of values from which the refractive index of the optical waveguide 4 is selected can be formed from any combination of the upper and lower refractive index bounds set out in the preceding paragraphs.

The optical waveguide/fibre 4 can be made from any suitable material or combination of materials. For example optical waveguides/fibres can be composed of or comprise silica, fluoride glass, phosphate glass, chalcogenide glass, and/or crown glass (such as BK7).

As noted above, in order to cut hair effectively, the optical waveguide 4 should ideally remain in contact with the same part of the hair for a prolonged period of time to allow coupled light to initiate the coupling and cut through the minimum amount of hair. However, since the refractive index of skin is close to the refractive index of hair, the laser light will also be able to couple into the skin if the optical waveguide 4 is brought into contact with the skin, and potentially burn or irritate the skin, particularly if the optical waveguide 4 remains on the same point of the skin for a prolonged period of time. Thus, according to the invention the optical waveguide 4 is adapted so that at least a portion of the cutting face 14 has high friction with hair (i.e. adheres or is 'sticky' to hair) to prolong the contact with the hair, and so that another portion of the optical waveguide 4 that can contact skin when the device 2 is in use has low friction with skin (i.e. does not adhere so much or is less 'sticky' to skin), to reduce the length of time that the optical waveguide 4 is in contact with a particular area of skin. In particular, the optical waveguide 4 is adapted so that the friction between at least a portion of the cutting face 14 and the hair is higher than between a conventional optical waveguide 4 and hair to increase the period of time in which the cutting face 14 is in contact with the hair. Furthermore, the optical waveguide 4 is adapted so that the friction between at least a portion of the optical waveguide 4 and the skin is lower than between a conventional optical waveguide 4 and skin to reduce the length of time that the optical waveguide 4 is in contact with a particular area of skin.

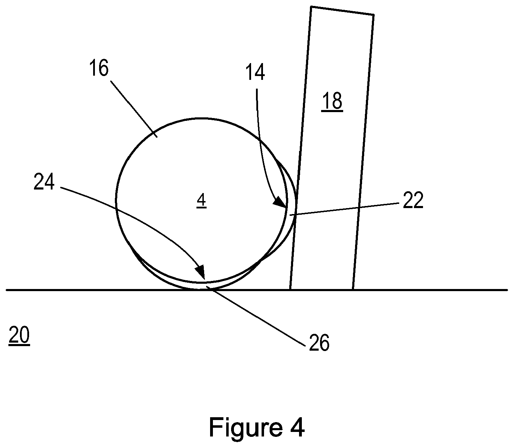

Fig. 4 illustrates an exemplary embodiment of the optical waveguide 4 according to the invention. The optical waveguide 4 is for use in or with a hair cutting device 2, for example as shown in Figs. 1 and 2. In Fig. 4 the optical waveguide 4 is shown side on (i.e. looking down the optical axis of the optical waveguide 4), and no other support element for the optical waveguide 4 is shown.

In Fig. 4, the optical waveguide 4 has a core 16. In this illustrated embodiment, the optical waveguide 4 does not include any cladding around the core 16. However it will be appreciated that in some embodiments the optical waveguide 4 can comprise cladding around the core 16, although preferably no cladding is present along the cutting face 14 (and indeed, in some embodiments the cutting face 14 can correspond to those parts of the optical waveguide 4 where there is no cladding).

The optical waveguide 4 is shown in contact with a hair 18 and the skin 20. The portion of the side wall of the core 16/optical waveguide 4 that is intended to contact hairs during use forms the cutting face 14. As described above, the refractive index of the core 16 is the same or lower than the refractive index of hair.

The core 16 may have a uniform refractive index (i.e. the same refractive index throughout the core 16), or it may be a graded index fibre, which means that the refractive index decreases with increasing distance from the optical axis.

In accordance with the invention, at least a portion of the cutting face 14 is adapted to provide higher friction (e.g. adhere or be 'sticky') to hair by providing at least a portion of the cutting face 14 with a layer or coating 22 that is lipophilic. That is, the layer or coating 22 (which is also referred to herein as a first layer or coating 22) is formed from or comprises a material that is lipophilic, i.e. a material that is soluble in fats, oils and lipids.

It will be understood that sebaceous glands in the skin secrete an oily or waxy substance called sebum, and the sebum covers the skin and hairs. Thus, the lipophilic material that forms the layer or coating 22 on the cutting face 14 is sticky to (i.e. adheres to) the sebum on the hair 18, which prolongs the time that the optical waveguide 4 is in contact with the hair 18 compared to a non-lipophilic material, and thereby increases the amount of light that couples into the skin. It will be understood that the lipophilic material effectively increases the friction between the optical waveguide 4 and the sebum on the hair 18 to prolong the contact time.

It will be appreciated that the layer or coating 22 can be applied to or cover just a portion of the cutting face 14 or all of the cutting face 14. In some embodiments the layer or coating 22 can also cover other parts of the side wall of the optical waveguide 4, but it will be appreciated that the first layer or coating 22 should not cover or be applied to a part of the side wall that can typically contact the skin 20 during use of the device 2 (as otherwise the layer or coating 22 will adhere or stick to the sebum on the skin 20).

It will also be appreciated that although the term 'lipophilic' is used herein, an alternative term that can be used to describe the desired properties of the first layer or coating 22 is 'oleophilic'.

The first layer or coating 22 can be formed from or comprise any lipophilic material, such as an oil, heavy hydro-carbons, fat, esters, petroleum by-products or derived products such as polyethene, polypropane, amino acids, or nickel silicon carbide.

It will be appreciated that the lipophilic material should be transparent or at least partially transparent (i.e. translucent) to light at the wavelengths used in the optical waveguide 4 to allow the light to couple to the hair through the layer or coating 22.

The layer or coating 22 can have any suitable thickness, for example between 0.01 microns and 100 microns, and more preferably a thickness between 0.1 microns and 10 microns.

It will be appreciated that the lipophilic material that forms the layer or coating 22 should have a refractive index that is equal to or higher than that of the optical waveguide 4 and a refractive index that is equal to or less than that of hair 18 to allow light to couple from the optical waveguide 4 to the hair 18 via the first layer or coating 22.

The lipophilic material can be a solid, or a fluid. It will also be appreciated that the lipophilic material should be biocompatible to avoid causing any adverse reaction or effect on the skin 20.

Also in accordance with the invention, the optical waveguide 4 is adapted to provide lower friction (e.g. be less adherent or be less 'sticky') to skin by providing at least a portion 24 of the side wall of the optical waveguide 4 with a layer or coating 26 that is lipophobic. The portion 24 is a part of the side wall of the optical waveguide 4 that can come into contact with the skin 20 when the device 2 is being used. Thus, the layer or coating 26 (which is also referred to herein as a second layer or coating 26) is formed from or comprises a material that is lipophobic, i.e. a material that is not soluble in fats, oils and lipids, and does not absorb fats, oils or lipids. Thus, the lipophobic material that forms the layer or coating 26 is not sticky to (i.e. not adherent to) the sebum on the skin 20, which allows the optical waveguide 4 to move more easily or smoothly over the skin 20 compared to a non-lipophobic material, and thereby reduces the contact time between the optical waveguide 4 and a particular area of skin 20. It will be understood that the lipophobic material effectively decreases the friction between the optical waveguide 4 and the sebum on the skin 20 to shorten the contact time with a particular area of skin 20.

It will be appreciated that the second layer or coating 26 can be applied to or cover just a portion of the side wall of the optical waveguide 4 that can come into contact with the skin 20 when the device 2 is being used. In alternative embodiments the second layer or coating 26 can be applied to or cover all of the parts of the side wall that can come into contact with the skin 20 when the device 2 is being used. In some embodiments the second layer or coating 26 can also cover other parts of the side wall of the optical waveguide 4 but it will be appreciated that the second layer or coating 26 should not cover or be applied to the cutting face 14 (as otherwise the second layer or coating 26 will reduce the stickiness or friction with the sebum on the hair 18.

It will also be appreciated that although the term 'lipophobic' is used herein, an alternative term that can be used to describe the desired properties of the second layer or coating 26 is 'oleophobic'.

The second layer or coating 26 can be formed from or comprise a lipophobic polymer material. For example, the second layer or coating 26 can be a polymer material such as polytetrafluoroethylene (PTFE). Alternatively, the second layer or coating 26 can be formed from or comprise a fluorinated compound, such as a fluoro-polymer, a polysiloxane or a fluorosilicone hybrid coating, or a so-called nano-coating.

In some embodiments, the lipophobic material that the second layer or coating 26 is formed from or comprises can be reflective to light at at least the wavelengths used in the optical waveguide 4 to reduce the possibility of light coupling into the skin 20.

The second layer or coating 26 can have any suitable thickness, for example between 0.1 microns and 2000 microns, and more preferably a thickness between 0.1 microns and 500 microns. A larger thickness for the second layer or coating 26 can enable the second layer or coating 26 to provide mechanical support to the optical waveguide 4.

In some embodiments, the lipophobic material in the second layer or coating 26 can have a refractive index that is lower than the refractive index of the optical waveguide 4 so that coupling of light to the second layer or coating 26 is prevented or reduced.

The lipophobic material can be a solid, or a fluid. It will also be appreciated that the lipophobic material should be biocompatible to avoid causing any adverse reaction or effect on the skin 20.

Therefore the modified optical waveguide 4 described above improves the cutting efficiency by prolonging contact with hair and reduces the contact time with a particular area of skin, thereby reducing the risk of causing damage or injury to the skin of the subject.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A hair cutting device for cutting hair on a body of a subject, the hair cutting device comprising:
a light source for generating laser light at one or more specific wavelengths corresponding to wavelengths absorbed by one or more chromophores in or on hair; and
a cutting element that comprises an optical waveguide that is coupled to the light source to receive laser light, wherein a portion of a sidewall of the optical waveguide forms a cutting face for contacting hair;
**characterised in that** at least a portion of the cutting face is provided with a first coating or layer that is formed from or comprises a lipophilic material; and wherein at least a portion of the sidewall of the optical waveguide that can contact the skin of the subject has a second coating or layer that is formed from or comprises a lipophobic material.

2. A hair cutting device as claimed in claim 1, wherein the first coating or layer is provided to increase friction between the optical waveguide and hair.

3. A hair cutting device as claimed in claim 1 or 2, wherein the second coating or layer is provided to reduce friction between the optical waveguide and skin.

4. A hair cutting device as claimed in claim 1, 2 or 3, wherein the lipophilic material is an oil, a heavy hydro-carbon, a fat, an ester, a petroleum by-product or derived product such as polyethene, polypropane, amino acids, or nickel silicon carbide.

5. A hair cutting device as claimed in any of claims 1-4, wherein the first coating or layer is transparent or partially transparent to light at one or more of said one or more specific wavelengths.

6. A hair cutting device as claimed in any of claims 1-5, wherein the first coating or layer has a thickness between 0.01 microns and 100 microns, or a thickness between 0.1 microns and 10 microns.

7. A hair cutting device as claimed in any of claims 1-6, wherein the first coating or layer has a refractive index that is equal to or higher than that of the optical waveguide and a refractive index that is equal to or lower than that of hair.

8. A hair cutting device as claimed in any of claims 1-7, wherein the lipophobic material is a polymer material.

9. A hair cutting device as claimed in any of claims 1-8, wherein the lipophobic material is polytetrafluoroethylene, PTFE, or a fluorinated compound.

10. A hair cutting device as claimed in any of claims 1-9, wherein the second coating or layer is reflective to light at one or more of said one or more specific wavelengths.

11. A hair cutting device as claimed in any of claims 1-10, wherein the second coating or layer has a thickness between 0.1 microns and 2000 microns, or a thickness between 0.1 microns and 500 microns.

12. A hair cutting device as claimed in any of claims 1-11, wherein the second coating or layer has a refractive index that is lower than that of the optical waveguide.

## Patentansprüche

1. Haarschneidevorrichtung zum Schneiden von Haaren an einem Körper eines Subjekts, wobei die Haarschneidevorrichtung umfasst:
eine Lichtquelle zum Erzeugen von Laserlicht bei einer oder mehreren spezifischen Wellenlängen, die Wellenlängen entsprechen, die von einem oder mehreren Chromophoren in oder auf Haaren absorbiert werden;
und ein Schneidelement, das einen optischen Wellenleiter umfasst, der mit der Lichtquelle gekoppelt ist, um Laserlicht zu empfangen, wobei ein Abschnitt einer Seitenwand des optischen Wellenleiters eine Schneidfläche zum Kontaktieren von Haaren bildet;
**dadurch gekennzeichnet, dass**
mindestens ein Abschnitt der Schneidfläche mit einer ersten Beschichtung oder Schicht versehen ist, die aus einem lipophilen Material gebildet ist oder dieses umfasst; und wobei mindestens ein Teil der Seitenwand des optischen Wellenleiters, der die Haut des Subjekts berühren kann, eine zweite Beschichtung oder Schicht aufweist, die aus einem lipophoben Material gebildet ist oder dieses umfasst.

2. Haarschneidevorrichtung nach Anspruch 1, wobei die erste Beschichtung oder Schicht bereitgestellt ist, um eine Reibung zwischen dem optischen Wellenleiter und dem Haar zu erhöhen.

3. Haarschneidevorrichtung nach Anspruch 1 oder 2, wobei die zweite Beschichtung oder Schicht bereitgestellt ist, um eine Reibung zwischen dem optischen Wellenleiter und der Haut zu verringern.

4. Haarschneidevorrichtung nach Anspruch 1, 2 oder 3, wobei das lipophile Material ein Öl, ein schwerer Kohlenwasserstoff, ein Fett, ein Ester, ein Erdölnebenprodukt oder ein abgeleitetes Produkt wie Polyethen, Polypropan, Aminosäuren oder Nickelsiliciumcarbid ist.

5. Haarschneidevorrichtung nach einem der Ansprüche 1 bis 4, wobei die erste Beschichtung oder Schicht für Licht bei einer oder mehreren der einen oder mehreren spezifischen Wellenlängen transparent oder teilweise transparent ist.

6. Haarschneidevorrichtung nach einem der Ansprüche 1 bis 5, wobei die erste Beschichtung oder Schicht eine Dicke zwischen 0,01 Mikrometer und 100 Mikrometer oder eine Dicke zwischen 0,1 Mikrometer und 10 Mikrometer aufweist.

7. Haarschneidevorrichtung nach einem der Ansprüche 1 bis 6, wobei die erste Beschichtung oder Schicht einen Brechungsindex, der gleich oder höher als der des optischen Wellenleiters ist, und einen Brechungsindex aufweist, der gleich oder niedriger als der des Haares ist.

8. Haarschneidevorrichtung nach einem der Ansprüche 1 bis 7, wobei das lipophobe Material ein Polymermaterial ist.

9. Haarschneidevorrichtung nach einem der Ansprüche 1 bis 8, wobei das lipophobe Material Polytetrafluorethylen, PTFE, oder eine fluorierte Verbindung ist.

10. Haarschneidevorrichtung nach einem der Ansprüche 1 bis 9, wobei die zweite Beschichtung oder Schicht für Licht bei einer oder mehreren der einen oder mehreren spezifischen Wellenlängen reflektierend oder teilweise reflektierend ist.

11. Haarschneidevorrichtung nach einem der Ansprüche 1 bis 10, wobei die zweite Beschichtung oder Schicht eine Dicke zwischen 0,1 Mikrometer und 2000 Mikrometer, oder eine Dicke zwischen 0,1 Mikrometer und 500 Mikrometer aufweist.

12. Haarschneidevorrichtung nach einem der Ansprüche 1 bis 11, wobei die zweite Beschichtung oder Schicht einen Brechungsindex aufweist, der niedriger als der des optischen Wellenleiters ist.

## Revendications

1. Dispositif de coupe de cheveux pour couper des cheveux sur un corps d'un sujet, le dispositif de coupe de cheveux comprenant :
une source de lumière pour générer une lumière laser à une ou plusieurs longueurs d'onde spécifiques correspondant à des longueurs d'onde absorbées par un ou plusieurs chromophores dans ou sur des cheveux ; et
un élément de coupe qui comprend un guide d'onde optique qui est couplé à la source de lumière pour recevoir de la lumière laser, dans lequel une partie d'une paroi latérale du guide d'onde optique forme une face de coupe pour entrer en contact avec des cheveux ;
**caractérisé en ce qu'**au moins une partie de la face de coupe est dotée d'un premier revêtement ou d'une première couche qui est formé à partir d'un matériau lipophile ou comprend celui-ci ; et dans lequel au moins une partie de la paroi latérale du guide d'onde optique qui peut entrer en contact avec la peau du sujet présente un second revêtement ou une seconde couche qui est formé à partir d'un matériau lipophobe ou comprend celui-ci.

2. Dispositif de coupe de cheveux selon la revendication 1, dans lequel le premier revêtement ou la première couche est prévu pour augmenter le frottement entre le guide d'onde optique et des cheveux.

3. Dispositif de coupe de cheveux selon la revendication 1 ou 2, dans lequel le second revêtement ou la seconde couche est prévu pour réduire le frottement entre le guide d'onde optique et la peau.

4. Dispositif de coupe de cheveux selon la revendication 1, 2 ou 3, dans lequel le matériau lipophile est une huile, un hydrocarbure lourd, une graisse, un ester, un sous-produit ou un dérivé du pétrole tel que le polyéthylène, le polypropane, des acides aminés ou du nickel-carbure de silicium.

5. Dispositif de coupe de cheveux selon l'une quelconque des revendications 1-4, dans lequel le premier revêtement ou la première couche est transparent ou partiellement transparent à la lumière à une ou plusieurs desdites une ou plusieurs longueurs d'onde spécifiques.

6. Dispositif de coupe de cheveux selon l'une quelconque de revendications 1-5, dans lequel le premier revêtement ou la première couche présente une épaisseur comprise entre 0,01 micron et 100 microns, ou une épaisseur comprise entre 0,1 micron et 10°micron.

7. Dispositif de coupe de cheveux selon l'une quelconque des revendications 1-6, dans lequel le premier revêtement ou la première couche présente un indice de réfraction qui est supérieur ou égal à celui du guide d'onde optique et un indice de réfraction qui est inférieur ou égal à celui des cheveux.

8. Dispositif de coupe de cheveux selon l'une quelconque des revendications 1-7, dans lequel le matériau lipophile est un matériau polymère.

9. Dispositif de coupe de cheveux selon l'une quelconque des revendications 1-8, dans lequel le matériau lipophobe est du polytétrafluoroéthylène, du PTFE ou un composé fluoré.

10. Dispositif de coupe de cheveux selon l'une quelconque des revendications 1-9, dans lequel le second revêtement ou la seconde couche réfléchit la lumière à une ou plusieurs desdites une ou plusieurs longueurs d'onde spécifiques.

11. Dispositif de coupe de cheveux selon l'une quelconque des revendications 1-10, dans lequel le second revêtement ou la seconde couche présente une épaisseur comprise entre 0,1 micron et 2 000 microns, ou une épaisseur comprise entre 0,1 micron et 500 microns.

12. Dispositif de coupe de cheveux selon l'une quelconque des revendications 1-11, dans lequel le second revêtement ou la seconde couche présente un indice de réfraction qui est inférieur à celui du guide d'onde optique.
